# EUROPEAN PATENT SPECIFICATION

(11) **EP 1 986 510 B1**
(45) Date of publication and mention of the grant of the patent: **21.11.2018**
(21) Application number: 06828739.0
(22) Date of filing: 15.12.2006
(51) Int. Cl.: A23L 33/16, A23L 2/52, A23C 9/15, A23C 9/152, A23C 9/20, A23L 2/74

(54) **MILK MINERAL WATER, PROCESSES FOR MANUFACTURING THEREOF AND BEVERAGES CONTAINING MILK MINERAL WATER**
MILCHMINERALWASSER, VERFAHREN ZU SEINER HERSTELLUNG UND MILCHMINERALWASSERHALTIGE GETRÄNKE
EAU MINERALE DE LAIT, PROCEDES POUR SA FABRICATION ET BOISSONS CONTENANT DE L'EAU MINERALE DE LAIT

(30) Priority: 15.12.2005 US 750401 P; 15.05.2006 US 800046 P; 21.07.2006 US 832159 P
(43) Date of publication of application: 05.11.2008
(73) Proprietor: Arla Foods Amba, 8260 Viby J (DK)
(72) Inventor: FRIIS, Torben, Lohse, 8260 Viby J (DK); HOLST, Hans, Henrik, 8260 Viby J (DK); JEPPESEN, Karsten, 8260 Viby J (DK); NYMARK, Katrine, 8260 Viby J (DK); SLOT, Carsten, Hallund, 8260 Viby J (DK); LAURITZEN, Karsten, 8260 Viby J (DK)
(74) Representative: Zacco Denmark A/S
(86) International application number: PCT/DK2006/000717
(87) International publication number: WO 2007/068253

(56) References cited:
- EP-A- 1 025 762
- EP-A1- 0 408 756
- EP-A1- 1 031 288
- EP-A2- 1 147 712
- WO-A-99/04903
- WO-A-99/40798
- WO-A-2004/019693
- GB-A- 2 130 069
- JP-B2- 2 924 914

## Description

### Field of the invention

The present invention relates to a milk mineral water in the form of a solely milk based liquid with characteristics of mineral water, processes for manufacturing thereof and a beverage containing such a milk mineral water.

### Background of the invention

There is a potential need for a drink which contains beneficial minerals from bovine milk, but without the energy input, flavour or colour that are natural for normal consumer's milk. This potential is illustrated by the well-documented fact that young people, as they grow older, switch from drinking milk to drinking water or soft drinks - without fulfilling their need for calcium.

Thus, there is great potential in succeeding to produce and commercialize a clear liquid or drink giving the same benefits as water (distribution, energy input, flavour, and taste) and simultaneously giving the consumer the calcium normally contained in consumer's milk.

WO 2004/019693 discloses a method for the production of a milk mineral water with reduced protein, fat and lactose level by using nanofiltration, ultrafiltration and reverse osmosis. The product can be regarded as a beverage (yoghurt drink).

### Summary of the invention

The invention is best described by the claims. The object of the invention is to produce a milk mineral water, where all the ingredients are dairy based. The liquid has sensory and shelf life properties similar to known bottled mineral water. It can be used in other beverages with different additives.

### Detailed description of the invention

The milk mineral water of the invention is a solely bovine milk based liquid with the characteristics of mineral water and having a calcium level between 0.1% and 200% of the calcium level of skimmed milk, a protein level between 0.05% and 2% of the normal protein level of skimmed milk, a fat level of between 0.01 % and 5% of the normal fat level of skimmed milk and a lactose level of between 0.01% and 4% of the normal lactose level of skimmed milk and produced by reverse osmosis of milk, sweet whey or acid whey, alternatively a milk, sweet whey or acid whey based UF permeate, wherein the reverse osmosis membrane has a pore size between 10⁻³ and 10⁻⁴ micron.

The energy level of such a liquid is between 0.01 and 10 kcal per 100 g.

It is normally desired to have the levels of protein, lactose and fat as low as practically possible due to the desired low energy level. However, there can be situations, where a certain content can be preferred due to the taste or dependant on optionally further additives and special utilisations.

Preferably the milk mineral water has a calcium level between 0.1 % and 120 % of the calcium level of skimmed milk, a protein level between 0.1% and 1.75% of the normal protein level of skimmed milk, a fat level between 0.01% and 4% of the normal fat level of skimmed milk and a lactose level between 0.01% and 2% of the normal lactose level of skimmed milk.

The energy level of such a milk mineral water is between 0.01 and 5 kcal per 100 g.

Another process for manufacturing of a liquid of claim 1 comprises nanofiltration of milk, sweet whey or acid whey, alternatively a milk, sweet whey or acid whey based ultrafiltration (UF) permeate mixed with either a milk, sweet whey or acid whey based evaporation condensate or alternatively a milk, sweet whey or acid whey based reverse osmosis (RO) permeate. It is also possible to combine such processes.

In all such processes a natural milk mineral concentrate can be added to the desired level, unless the level already is as preferred. "Capolac" from Aria Foods, Denmark can be used.

Further details of the manufacturing processes can be found in the examples. A milk mineral water of claim 1 can be used directly as mineral water or it can be used as essentially sole liquid combined with one or more of the following ingredients in beverages: a natural milk mineral concentrate, a food grade acid, a food grade sweetener, a food grade colorant, a food grade flavouring agent, a fruit juice, a fruit pulp, vitamins, plant extracts and carbonization.

A beverage containing a milk mineral water of claim 1 as sole liquid can be a soft drink, a food supplement or a natural medicinal product.

Manufacturing of the milk mineral water can thus be performed by
- Producing a permeate from reverse osmosis of milk, sweet whey or acid whey, alternatively a milk, sweet whey or acid whey based ultrafiltration (UF) permeate.

Furthermore, as mentioned above, one or more of the following ingredients may be added: a natural milk mineral concentrate, a food grade acid, a food grade sweetener, a food grade colorant, a food grade flavouring agent, a fruit juice, a fruit pulp, vitamins, plant extracts and carbonization.

The process is to concentrate milk, sweet whey, acid whey or ultrafiltration permeate from milk, sweet whey or acid whey on a nanofiltration membrane with a membrane pore size between 10⁻³ micron and 10⁻² micron, alternatively on a reverse osmosis membrane with a membrane pore size between 10⁻⁴ micron and 10⁻³ micron
Thereby, a clear liquid is obtained.

The liquid can thereafter, optionally after addition of milk mineral condensate or other additives, be heat-treated, e.g. in normally dairy equipment for heat-treatment of milk products.

Condensate from milk, sweet whey or acid whey evaporation may be added in a ratio of between 30 % and 70 %.

Furthermore the obtained liquid can be submitted to one or more of the following treatments:
- addition of a food grade flavour
- pH adjustment with a food grade acid, preferably lactic acid or citric acid, to pH 7.0 - 3.0 (7.0, 6.5, 6.0, 5.5, 5.0, 4.5, 4.0, 3.5, 3.0)
- addition of a natural milk mineral concentrate (e.g. Capolac from Aria Foods) to achieve a calcium level of 0.1%-200% (0.1%, 0.5% 1%, 5%, 10%, 25%, 50%, 75%, 100%, 125%, 150%, 175% or 200%) of the natural level of skimmed milk.

The normal content of calcium in milk is about 116 mg/100 g for whole milk (between 63 mg/100 g and 149 mg/100 g due to variations in fat content from animal to animal), whereas the content in skim milk is about 124 mg/100 g (with a natural variation of 114 - 134 mg/100g). Therefore skimmed milk is chosen as standard in the present application. It is possible to add more natural milk mineral concentrate. However, addition of too much will negatively affect the product taste.

The mineral water of the invention, solely based on dairy products, can be used in a beverage in the form of a soft drink, a food supplement, including a probiotic or prebiotic food supplement, or a natural medicinal product.

For such a beverage one or more of the following additives can be used:
- a food grade bulk sweetener
- a food grade high intensity sweetener
- a food grade colorant
- a fruit pulp
- a probiotic
- a prebiotic
- dairy based proteins, peptides and amino acids
- vegetable based proteins, peptides and amino acids
- vitamins
- dietary supplement e.g. ginseng, taurin, gingko biloba, green tea and aloe vera

The above mentioned list is a non limiting list, not excluding other additives.

This final product is heat-treated, preferably HTST pasteurized, followed by filling/closing.

### Abbreviations and definitions:

TS = Total Solids, equal to DM = Dry Matter
NF = Nanofiltration
UF = Ultrafiltration
RO = Reverse Osmosis
Sweet whey = whey with pH above pH 5.6
Acid whey = whey with pH below pH 5.1
Casein whey = whey from production of casein
HTST = high temperature short time
Milk mineral water = mineral water based solely on dairy products, such as milk, skimmed milk, sweet whey and acid whey as well as ultrafiltration (UF) permeates thereof.

Sole liquid = essentially sole liquid. This term is used for beverages of the invention and means that the beverage does not contain another liquid than the milk mineral water except for minor amounts of liquid from added extracts, flavours or the like additives.

Probiotics = live microorganisms which, when administered in adequate amounts, confer a health benefit on the host.

Prebiotics = food substances which promote the growth of certain bacteria (generally probiotics) in the intestines.

Absorbance= absorbance measured in a 1 cm. cuvette at 500 nm. Reference: demineralised water.

The present invention is further explained in the following non limiting examples.

The drawing shows the flow sheets for each example:
Fig 1 is the flow sheet for example 1.
Fig 2 is the flow sheet for example 2.
Fig 3 is the flow sheet for example 3.
Fig 4 is the flow sheet for example 4.
Fig 5 is the flow sheet for example 5.
Fig 6 is the flow sheet for example 6.
Fig 7 is the flow sheet for example 7.
Fig 8 is the flow sheet for example 8.
Fig 9 is the flow sheet for example 9.
Fig 10 is the flow sheet for example 10.

### Example 1:

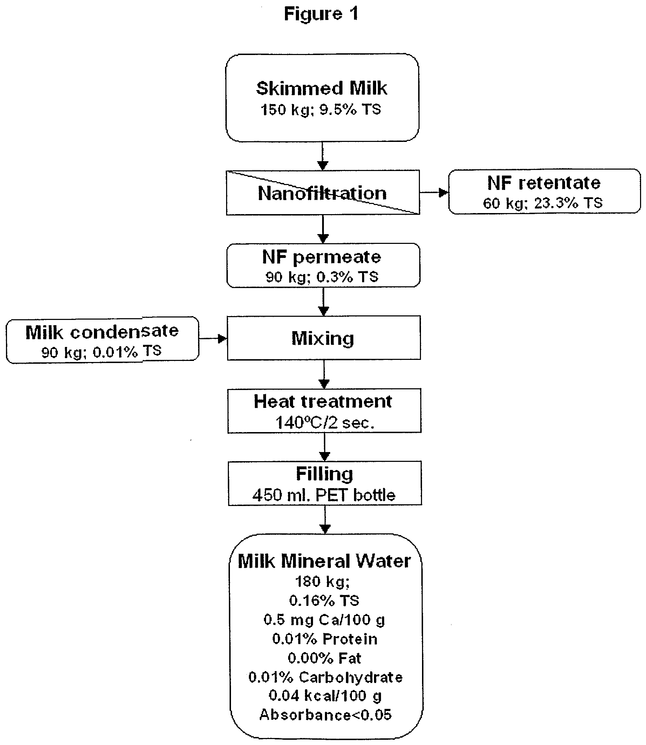

### (Reference example, out of the scope of the invention) Ref.: Figure 1

- 150 kg of skimmed milk (9.5% TS) are processed on a filtration unit equipped with NF membranes (Osmonics; cut-off: 200 MW). The concentration ratio is 1:2.5, resulting in 60 kg of nanofiltration retentate (23.3% TS) and 90 kg of NF permeate (0.3% TS).
- The 90 kg of NF permeate are mixed with 90 kg of condensate from skimmed milk evaporation (0.01% TS), taken from the first stage in a 3-stage evaporator from Anhydro A/S.
- The above mentioned liquid is heat-treated at 140 °C for 2 seconds, followed by filling into clear PET bottles, each containing 450 ml.

### Example 2:

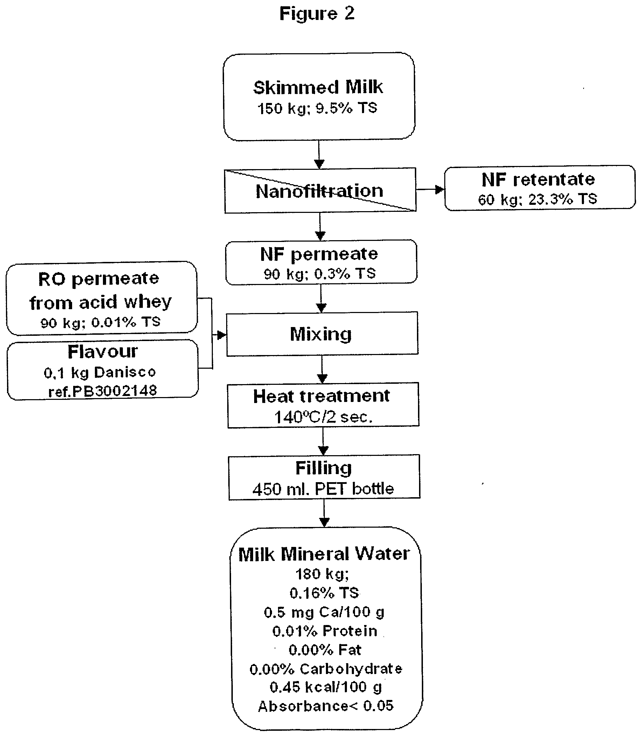

### (Reference example, out of the scope of the invention) Ref.: Figure 2

- 150 kg of skimmed milk (9.5% TS) are processed on a filtration unit equipped with NF membranes (Osmonics; cut-off: 200 MW). The concentration ratio is 1:2.5, resulting in 60 kg of NF retentate (23.3% TS) and 90 kg of NF permeate (0.3% TS).
- The 90 kg of NF permeate are mixed with 90 kg of RO permeate from reverse osmosis of acid casein whey (0.01 % TS).
- Then a flavour compound (0.1 kg. Lime, ref. PB3002148 from Danisco Ingredients) is added.
- Finally, the above mentioned liquid is heat-treated at 140 °C for 2 seconds, followed by filling into clear PET bottles, each containing 450 ml.

### Example 3:

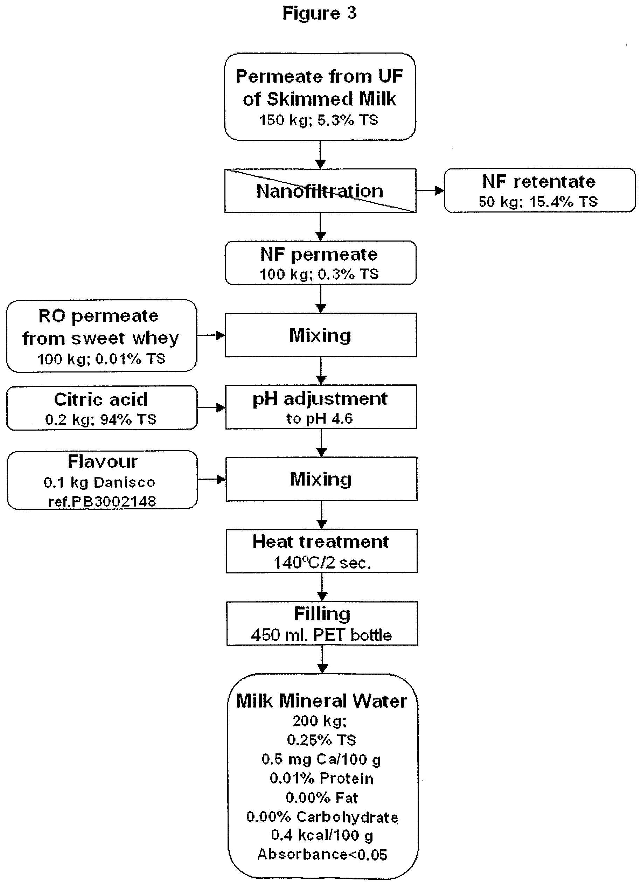

### Ref.: Figure 3

- 150 kg of UF permeate from skimmed milk (5.3% TS) was processed on a filtration unit equipped with NF membranes (Osmonics; cut-off: 200 MW). The concentration ratio was 1:3, resulting in 50 kg of NF retentate (15.4% TS) and 100 kg of NF permeate (0.3 %TS).
- The 100 kg of NF permeate was mixed with 100 kg of RO permeate from reverse osmosis of sweet whey (0.01% TS).
- The above mentioned mix of NF permeate and RO permeate was pH adjusted by adding citric acid, until pH 4.6 was achieved.
- Then a flavour compound (0.1 kg. Lime, ref. PB3002148 from Danisco Ingredients) was added.

Finally, the above mentioned liquid was heat-treated at 140 °C for 2 seconds, followed by filling into clear PET bottles, each containing 450 ml.

### Example 4:

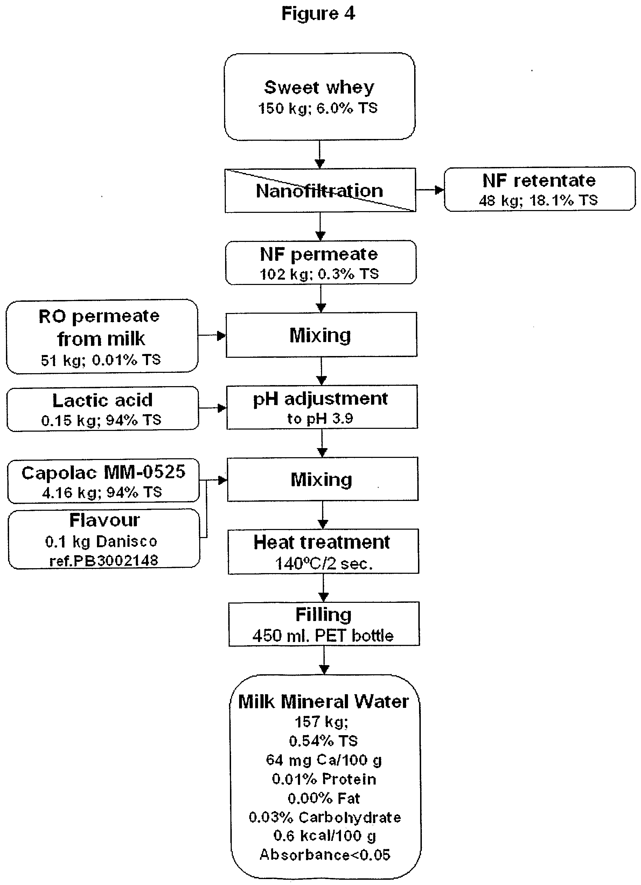

### Ref.: Figure 4

- 150 kg of sweet whey (6.0% TS) are processed on a filtration unit equipped with NF membranes (Osmonics; cut-off: 200 MW). The concentration ratio is 1:3, resulting in 48 kg of NF retentate (18.1% TS) and 102 kg of NF permeate (0.3% TS).
- The 102 kg of NF permeate are mixed with 51 kg of RO permeate from reverse osmosis of skimmed milk (0.01 % TS).
- The above mentioned mix of NF permeate and RO permeate is pH adjusted by adding lactic acid, until pH 3.9 is achieved.
- The next step is to dissolve 4.16 kg of milk calcium concentrate (Capolac MM0525 from Aria Foods) into the pH adjusted liquid.
- Then a flavour compound (0.1 kg. Lime, ref. PB3002148 from Danisco Ingredients) is added.

Finally, the above mentioned liquid is heat-treated at 140 °C for 2 seconds, followed by filling into clear PET bottles, each containing 450 ml.

### Example 5:

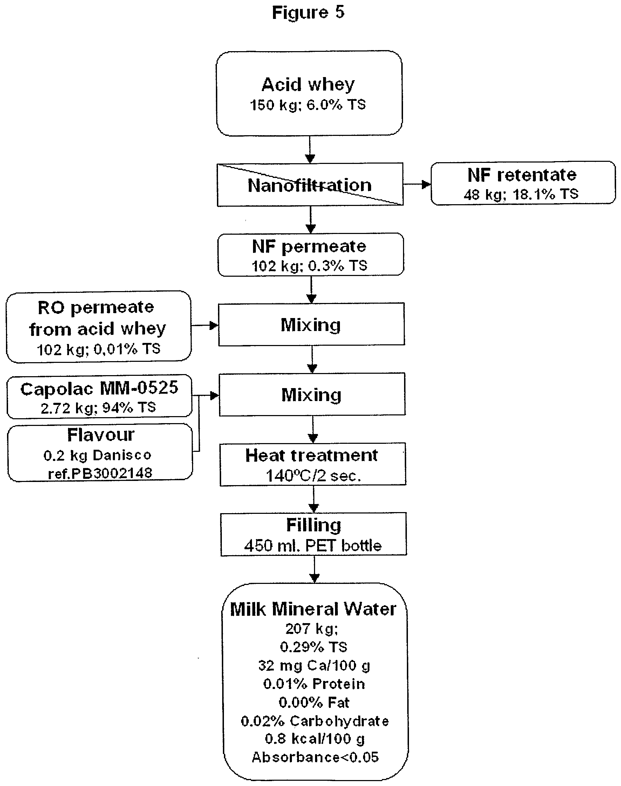

### Ref.: Figure 5

- 150 kg of acid whey (6.0% TS) are processed on a filtration unit equipped with NF membranes (Osmonics; cut-off: 200 MW). The concentration ratio is 1:3, resulting in 48 kg of NF retentate (18.1% TS) and 102 kg of NF permeate (0.3% TS).
- The 102 kg of NF permeate are mixed with 102 kg of RO permeate from reverse osmosis of acid whey (0.01 % TS).
- The next step is to dissolve 2.72 kg of milk calcium concentrate (Capolac MM0525 from Aria Foods) into the liquid.
- Then a flavour compound (0.2 kg. Lime, ref. PB3002148 from Danisco Ingredients) is added.
- Finally, the above mentioned liquid is heat-treated at 140 °C for 2 seconds, followed by filling into clear PET bottles, each containing 450 ml.

### Example 6:

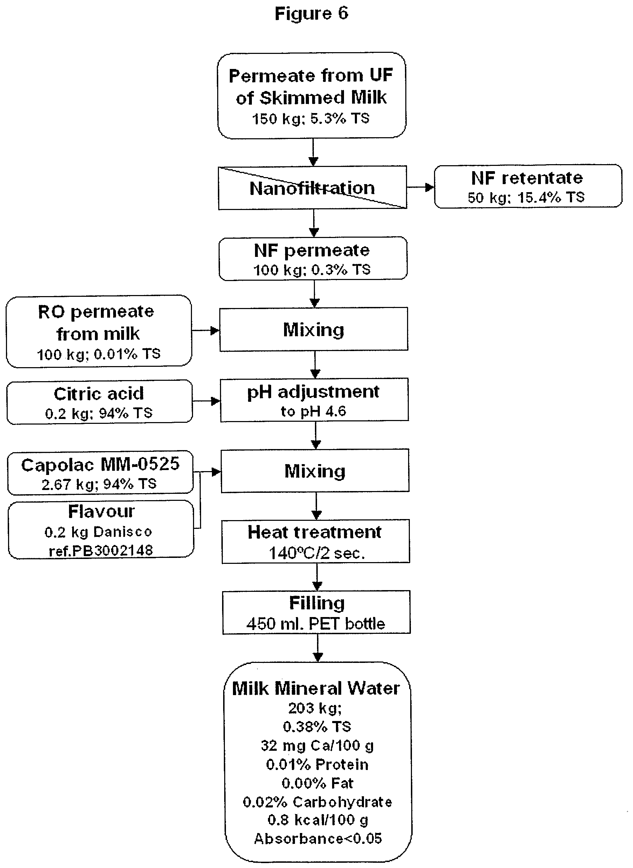

### Ref.: Figure 6

- 150 kg of UF permeate from ultrafiltration of skimmed milk (5.3% TS) was processed on a filtration unit, equipped with NF membranes (Osmonics; cut-off: 200 MW). The concentration ratio was 1:3, resulting in 50 kg of NF retentate (15.4 %TS) and 100 kg of NF permeate (0.3% TS).
- The 100 kg of NF permeate was mixed with 100 kg of RO permeate from reverse osmosis of skimmed milk (0.01 % TS).
- The above mentioned mix of NF permeate and RO permeate was pH adjusted by adding citric acid, until pH 4.6 was achieved.
- The next step was to dissolve 2.67 kg of milk calcium concentrate (Capolac MM0525 from Aria Foods) into the pH adjusted liquid.
- Then a flavour compound (0.2 kg. Lime, ref. PB3002148 from Danisco Ingredients) was added.

Finally, the above mentioned liquid was heat-treated at 140 °C for 2 seconds, followed by filling into clear PET bottles, each containing 450 ml.

### Example 7:

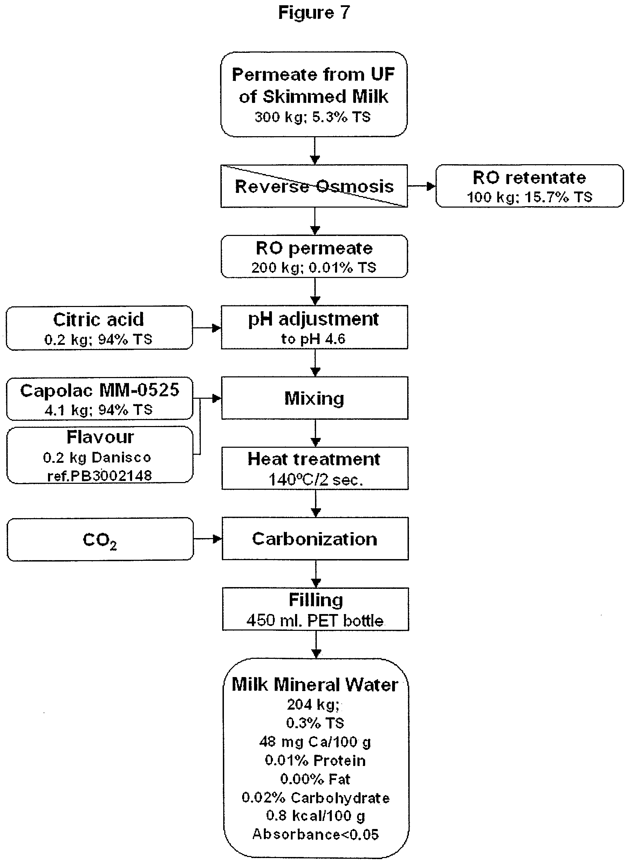

### Ref.: Figure 7

- 300 kg of UF permeate from ultrafiltration of skimmed milk (5.3% TS) was processed on a filtration unit, equipped with RO membranes (Osmonics; cut-off: 50 MW). The concentration ratio was 1:3, resulting in 100 kg of RO retentate (15.7% TS) and 200 kg of RO permeate (0.01 % TS).
- The 200 kg of RO permeate was pH adjusted by adding citric acid, until pH 4.6 was achieved.
- The next step was to dissolve 4.1 kg of milk calcium concentrate (Capolac MM0525 from Aria Foods) into the pH adjusted liquid.
- Then a flavour compound (0.2 kg. Lime, ref. PB3002148 from Danisco Ingredients) was added.

Finally, the above mentioned liquid was heat-treated at 140 °C for 2 seconds, followed by carbonisation and filling into clear PET bottles, each containing 450 ml.

### Example 8:

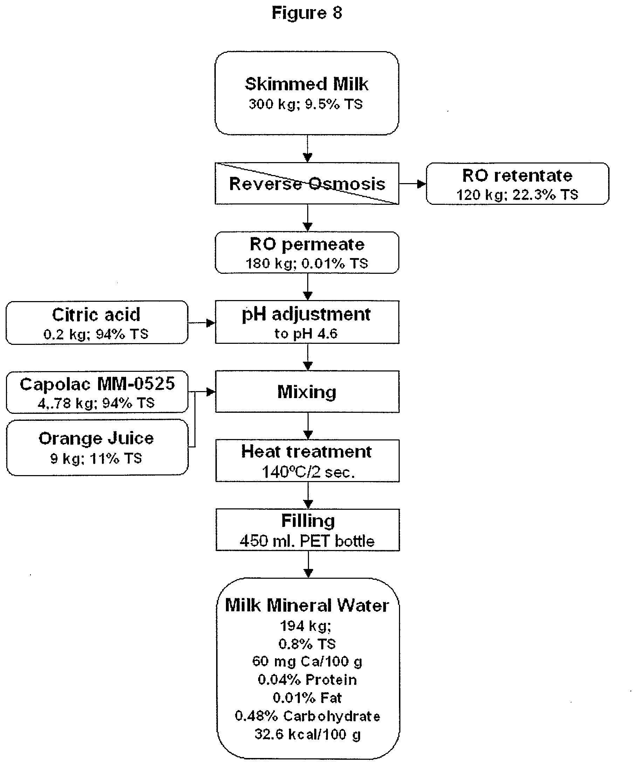

### Ref.: Figure 8

- 300 kg of skimmed milk (9.5% TS) are processed on a filtration unit equipped with RO membranes (Osmonics; cut-off: 50 MW). The concentration ratio is 1:2.5, resulting in 120 kg of RO retentate (22.3% TS) and 180 kg of RO permeate (0.01 % TS).
- The above mentioned RO permeate is pH adjusted by adding citric acid, until pH 4.6 is achieved.
- The next step is to dissolve 4.78 kg of milk calcium concentrate (Capolac MM0525 from Arla Foods) into the pH adjusted liquid.
- 9 kg of orange juice are mixed in.
- Finally, the above mentioned liquid is heat-treated at 140 °C for 2 seconds, followed by filling into clear PET bottles, each containing 450 ml.

### Example 9:

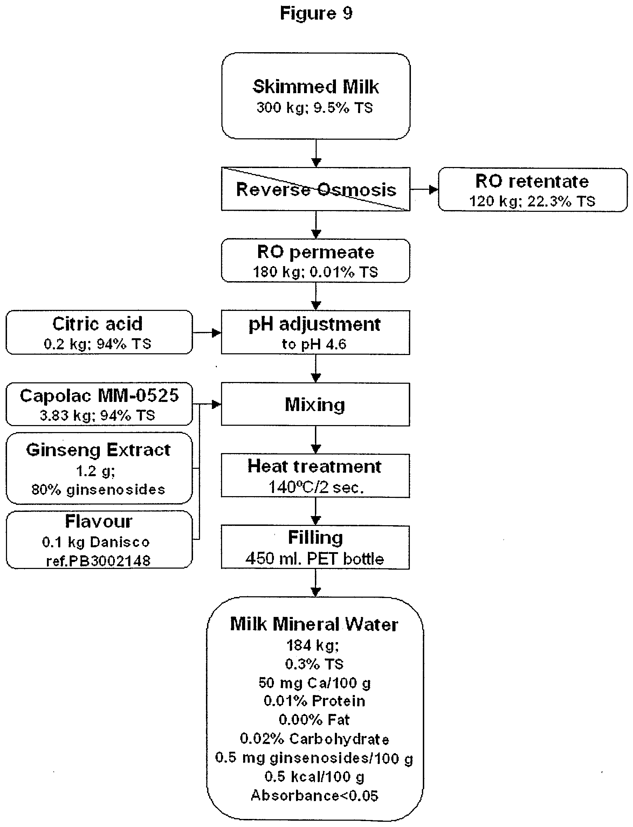

### Ref.: Figure 9

- 300 kg of skimmed milk (9.5% TS) are processed on a filtration unit equipped with RO membranes (Osmonics; cut-off: 50 MW). The concentration ratio is 1:2.5, resulting in 120 kg of RO retentate (22.3% TS) and 180 kg of RO permeate (0.01 % TS).
- The above mentioned RO permeate is pH adjusted by adding citric acid, until pH 4.6 is achieved.
- The next step is to dissolve 3.83 kg of milk calcium concentrate (Capolac MM0525 from Aria Foods) into the pH adjusted liquid.
- Then a flavour compound (0.1 kg. Lime, ref. PB3002148 from Danisco Ingredients) plus a ginseng extract (1.2 g; 80% ginsenosides) is added.
- Finally, the above mentioned liquid is heat-treated at 140 °C for 2 seconds, followed by filling into clear PET bottles, each containing 450 ml.

### Example 10:

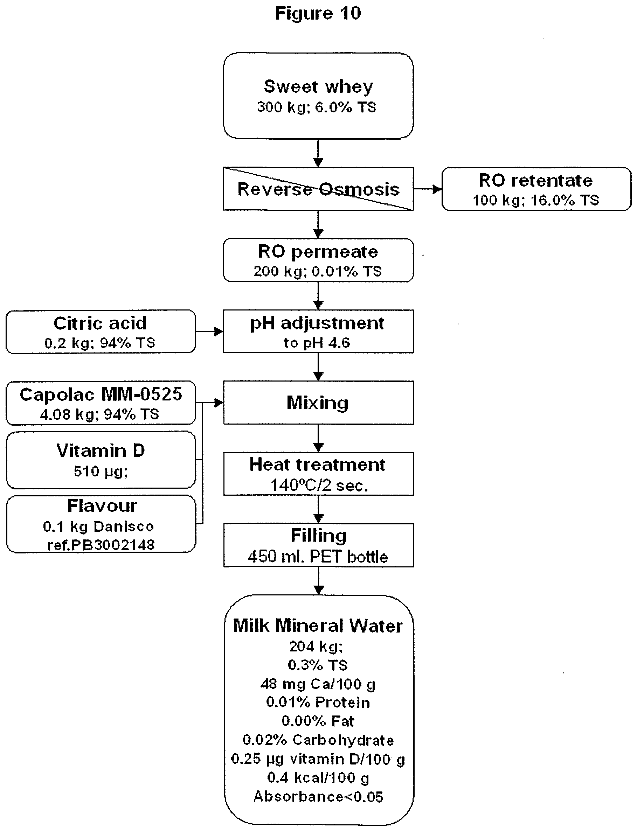

### Ref.: Figure 10

- 300 kg of sweet whey (6.0% TS) are processed on a filtration unit equipped with RO membranes (Osmonics; cut-off: 50 MW). The concentration ratio is 1:3, resulting in 100 kg of RO retentate (16.0% TS) and 200 kg of RO permeate (0.01 % TS).
- The above mentioned RO permeate is pH adjusted by adding citric acid, until pH 4.6 is achieved.
- The next step is to dissolve 4.08 kg of milk calcium concentrate (Capolac MM0525 from Aria Foods) into the pH adjusted liquid.
- Then a flavour compound (0.2 kg. Lime, ref. PB3002148 from Danisco Ingredients) plus a vitamin D extract (510 mg; 80% ginsenosides) is added.
- Finally, the above mentioned liquid is heat-treated at 140 °C for 2 seconds, followed by filling into clear PET bottles, each containing 450 ml.

## Claims

1. Process for manufacturing of a milk mineral water solely based on dairy products, wherein the calcium level is between 0.1 % and 200% of the calcium level of skimmed milk, the protein level is between 0.05% and 2% of the normal protein level of skimmed milk, the fat level is between 0.01 % and 5% of the normal fat level of skimmed milk, and the lactose level is between 0.01% and 4% of the level of skimmed milk, by reverse osmosis of milk, sweet whey or acid whey, alternatively a milk, sweet whey or acid whey based UF permeate, wherein the reverse osmosis membrane has a pore size between 10⁻⁴ and 10⁻³ micron.

2. The process of claim 1 wherein the calcium level is between 0.114 and 268 mg/100g.

3. The process of any of the claims 1 and 2 using reverse osmosis of a milk, sweet whey or acid whey based UF permeate.

4. The process of any of the claims 1 to 3 for manufacturing of a milk mineral water, wherein the calcium level is between 0.1 % and 120 % of the calcium level of skimmed milk, the protein level is between 0.1 % and 1.75% of the normal protein level of skimmed milk, the fat level is between 0.01 % and 4% of the normal fat level of skimmed milk and the lactose level is between 0.01 % and 2% of the normal lactose level of skimmed milk.

5. The process of any of the claims 1 to 4, further comprising mixing of the resulting reverse osmosis permeate with a permeate from nanofiltration of milk, sweet whey or acid whey, alternatively a milk, sweet whey or acid whey based ultrafiltration permeate.

6. The process of any of the claims 1 to 5, further comprising pH adjustment in the resulting reverse osmosis permeate with a food grade acid to pH between 7.0 and 3.0.

7. The process of claim 6, wherein the food grade acid is lactic acid.

8. The process of any of the claims 1 to 7, further comprising addition to the resulting reverse osmosis permeate of a food grade flavour.

9. The process of any of the claims 1 to 8, further comprising addition to the resulting reverse osmosis permeate of a natural milk mineral concentrate to achieve a calcium level of 0.1 to 200 % of skimmed milk.

10. The process of any of the claims 1 to 9, further comprising addition to the resulting reverse osmosis permeate of calcium from milk or another dairy product to the desired level in the beverage.

11. The process of any of the claims 1 to 10, further comprising heat-treatment of the resulting reverse osmosis permeate, optionally after addition of additives.

12. Process to prepare a beverage containing a milk mineral water, which process comprises preparation of a milk mineral water according to any of the claims 1 to 11, and addition of one or more of the following ingredients: a natural milk mineral concentrate, a food grade acid, a food grade sweetener, a food grade, a food grade colorant, a fruit grade flavouring agent, a fruit juice, a fruit pulp, a probiotic, a prebiotic, dairy based proteins, peptides and amino acids, vegetable based proteins, peptides and amino acids, vitamins, plant extracts and dietary supplement.

13. Process according to claim 12 wherein the beverage comprises addition of carbonisation.

14. Process according to any of the claims 12 to 13, wherein the beverage is as a soft drink, a food supplement or a natural medicinal product.

15. Process according to claim 14, wherein the food supplement is a probiotic or prebiotic food supplement.

## Patentansprüche

1. Verfahren zur Herstellung eines Milchmineralwassers, das lediglich auf Milchprodukten basiert, wobei der Calciumgehalt zwischen 0,1 % und 200 % des Calciumgehalts von Magermilch beträgt, der Proteingehalt zwischen 0,05 % und 2 % des normalen Proteingehalts von Magermilch beträgt, der Fettgehalt zwischen 0,01 % og 5 % des normalen Fettgehalts von Magermilch beträgt, und der Lactosegehalt zwischen 0,01 % og 4 % des Gehalts von Magermilch beträgt, durch Umkehrosmose von Milch, Süß- oder Sauermolke, alternativ von einem auf Milch, Süß- oder Sauermolke basierenden UF-Permeat, wobei die Umkehrosmosemembran eine Porengröße von zwischen 10⁻⁴ und 10⁻³ Mikron aufweist.

2. Verfahren nach Anspruch 1, wobei der Calciumgehalt zwischen 0,114 und 268 mg/100g beträgt.

3. Verfahren nach einem der Ansprüche 1 und 2 unter Anwendung von Umkehrosmose eines auf Milch, Süß- oder Sauermolke basierenden UF-Permeats.

4. Verfahren nach einem der Ansprüche 1 bis 3 zur Herstellung eines Milchmineralwassers, wobei der Calciumgehalt zwischen 0,1 % und 120 % des Calciumgehalts von Magermilch beträgt, der Proteingehalt zwischen 0,1 % und 1,75% des normalen Proteingehalts von Magermilch beträgt, der Fettgehalt zwischen 0,01 % und 4 % des normalen Fettgehalts von Magermilch beträgt, und der Lactosegehalt zwischen 0,01 % und 2 % des normalen Lactosegehalts von Magermilch beträgt.

5. Verfahren nach einem der Ansprüche 1 bis 4, ferner umfassend ein Vermischen des sich ergebenden Umkehrosmosepermeats mit einem Permeat aus Nanofiltration von Milch, Süß- oder Sauermolke, alternativ einem auf Milch, Süß- oder Sauermolke basierenden Ultrafiltrationspermeat.

6. Verfahren nach einem der Ansprüche 1 bis 5, ferner umfassend ein pH-Einstellen des sich ergebenden Umkehrosmosepermeats mit einer Säure von Lebensmittelqualität auf einen pH-Wert von zwischen 7,0 und 3,0.

7. Verfahren nach Anspruch 6, wobei die Säure von Lebensmittelqualität Milchsäure ist.

8. Verfahren nach einem der Ansprüche 1 bis 7, ferner umfassend die Zugabe zu dem sich ergebenden Umkehrosmosepermeat eines Geschmacks von Lebensmittelqualität.

9. Verfahren nach einem der Ansprüche 1 bis 8, ferner umfassend die Zugabe zu dem sich ergebenden Umkehrosmosepermeat eines natürlichen Milchmineralkonzentrats, um einen Calciumgehalt von 0,1 bis 200 % von Magermilch zu erhalten.

10. Verfahren nach einem der Ansprüche 1 bis 9, ferner umfassend die Zugabe zu dem sich ergebenden Umkehrosmosepermeat von Calcium aus Milch oder einem anderen Milchprodukt bis zu der gewünschten Ebene in dem Getränk.

11. Verfahren nach einem der Ansprüche 1 bis 10, ferner umfassend die Wärmebehandlung des sich ergebenden Umkehrosmosepermeats, wahlweise nach der Zugabe von Zusatzstoffen.

12. Verfahren zur Herstellung eines ein Milchmineralwasser enthaltenden Getränks, wobei das Verfahren die Herstellung eines Milchmineralwassers nach einem der Ansprüche 1 bis 11, und die Zugabe einer oder mehrerer der folgenden Zutaten umfasst: ein natürliches Milchmineralkonzentrat, eine Säure von Lebensmittelqualität, einen Süßstoff von Lebensmittelqualität, eine Lebensmittelqualität, einen Farbstoff von Lebensmittelqualität, eine Fruchtsaft, ein Fruchtmark, ein Probiotikum, ein Präbiotikum, auf Milchprodukte basierende Proteine, Peptide und Aminosäuren, Proteine, Peptide und Aminosäuren pflanzlichen Ursprungs, Vitamine, Pflanzextrakte und Nahrungsergänzungsmittel.

13. Verfahren nach Anspruch 12, wobei das Getränk die Zugabe von Carbonisierung umfasst.

14. Verfahren nach einem der Ansprüche 12 bis 13, wobei das Getränk ein Erfrischungsgetränk, ein Nahrungsergänzungsmittel oder ein natürliches medizinisches Produkt ist.

15. Verfahren nach Anspruch 14, wobei das Nahrungsergänzungsmittel ein probiotisches oder präbiotisches Nahrungsergänzungsmittel ist.

## Revendications

1. Procédé de fabrication d'une eau minérale de lait uniquement à base de produits laitiers, dans lequel le taux de calcium est compris entre 0,1 % et 200% du taux de calcium du lait écrémé, le taux de protéines est compris entre 0,05% et 2% du taux de protéines normal du lait écrémé, le taux de matière grasse est compris entre 0,01 % et 5% du taux de matière grasse normale du lait écrémé, et le taux de lactose est compris entre 0,01% et 4% du taux de lait écrémé, par l'osmose inverse de lait, de lactosérum doux ou de lactosérum acide, en variante par un perméat d'ultrafiltration à base de lait, de lactosérum doux ou de lactosérum acide, la membrane d'osmose inverse présentant une taille de pores comprise entre 10⁻⁴ et 10⁻³ microns.

2. Procédé selon la revendication 1, dans lequel le taux de calcium est compris entre 0,114 et 268 mg/100g.

3. Procédé selon l'une quelconque des revendications 1 et 2, utilisant l'osmose inverse d'un perméat d'ultrafiltration à base de lait, de lactosérum doux ou de lactosérum acide.

4. Procédé selon l'une quelconque des revendications 1 à 3 de fabrication d'une eau minérale de lait, dans laquelle le taux de calcium est compris entre 0,1 % et 120 % du taux de calcium du lait écrémé, le taux de protéines est compris entre 0,1 % et 1,75% du taux de protéines normal du lait écrémé, le niveau de matière grasse est compris entre 0,01 % et 4% du taux de matière grasse normal du lait écrémé et le taux de lactose est compris entre 0,01 % et 2% du taux de lactose normal du lait écrémé.

5. Procédé selon l'une quelconque des revendications 1 à 4, comprenant en outre le mélange du perméat d'osmose inverse résultant avec un perméat issu de la nano-filtration de lait, de lactosérum doux ou de lactosérum acide, en variante d'un perméat d'ultrafiltration à base de lait, de lactosérum doux ou de lactosérum acide.

6. Procédé selon l'une quelconque des revendications 1 à 5, comprenant en outre l'ajustement de la valeur pH dans le perméat d'osmose inverse résultant avec un acide de qualité alimentaire à une valeur de pH comprise entre 7,0 et 3,0.

7. Procédé selon la revendication 6, dans lequel l'acide de qualité alimentaire est de l'acide lactique.

8. Procédé selon l'une quelconque des revendications 1 à 7, comprenant en outre l'addition au perméat d'osmose inverse résultant d'une flaveur de qualité alimentaire.

9. Procédé selon l'une quelconque des revendications 1 à 8, comprenant en outre l'addition au perméat d'osmose inverse résultant d'un concentré minéral de lait naturel pour obtenir un taux de calcium de 0,1 à 200 % de lait écrémé.

10. Procédé selon l'une quelconque des revendications 1 à 9, comprenant en outre l'addition au perméat d'osmose inverse résultant de calcium provenant de lait ou d'un autre produit laitier jusqu'au taux désiré dans la boisson.

11. Procédé selon l'une quelconque des revendications 1 à 10, comprenant en outre le traitement thermique du perméat d'osmose inverse résultant, éventuellement après l'addition d'additifs.

12. Procédé de préparation d'une boisson contenant une eau minérale de lait, le procédé comprenant la préparation d'une eau minérale de lait selon l'une quelconque des revendications 1 à 11, et l'addition de l'un ou de plusieurs des ingrédients suivants : un concentré minéral de lait naturel, un acide de qualité alimentaire, un édulcorant de qualité alimentaire, une qualité alimentaire, un colorant de qualité alimentaire, un agent d'aromatisation de qualité fruitière, un jus de fruit, une pulpe de fruit, un probiotique, un prébiotique, des protéines, des peptides et des acides aminés à base de produits laitiers, des protéines, des peptides et des acides aminés à base végétale, des vitamines, des extraits de plantes et complément diététique.

13. Procédé selon la revendication 12, dans lequel la boisson comprend l'addition de la carbonisation.

14. Procédé selon l'une quelconque des revendications 12 à 13, dans lequel la boisson est une boisson non alcoolisée, un complément alimentaire ou un médicament naturel.

15. Procédé selon la revendication 14, dans lequel le complément alimentaire est un complément alimentaire probiotique ou prébiotique.
